# EUROPÄISCHE PATENTSCHRIFT

(11) **EP 2 892 608 B1**
(45) Veröffentlichungstag und Bekanntmachung des Hinweises auf die Patenterteilung: **02.10.2019**
(21) Anmeldenummer: 13745070.6
(22) Anmeldetag: 02.08.2013
(51) Int. Cl.: A61M 39/10, A61M 39/12

(54) **KATHETERKUPPLUNG**
CATHETER COUPLING
RACCORD DE CATHÉTER

(30) Priorität: 17.08.2012 DE 202012007845 U
(43) Veröffentlichungstag der Anmeldung: 15.07.2015
(73) Patentinhaber: Raumedic AG, 95213 Münchberg (DE); B. Braun Melsungen AG, 34212 Melsungen (DE)
(72) Erfinder: KELLNER, Thorsten, 95445 Bayreuth (DE); SKAPER, Frank, 95191 Leupoldsgrün (DE); JAKOB, Thomas, 95111 Rehau (DE); BEZELA, Manuela, 34326 Morschen (DE); BLUM, Iris, 34277 Fuldabrück (DE); SIPPEL, Martin, 34212 Melsungen (DE); WIEGEL, Heinz, 36211 Alheim (DE)
(74) Vertreter: Rau, Schneck & Hübner Patentanwälte Rechtsanwälte PartGmbB
(86) Internationale Anmeldenummer: PCT/EP2013/066277
(87) Internationale Veröffentlichungsnummer: WO 2014/026864

(56) Entgegenhaltungen:
- EP-B1- 1 033 146
- DE-A1-102007 029 229
- DE-U1- 8 425 441
- DE-U1- 9 015 688

## Beschreibung

Der Inhalt der deutschen Gebrauchsmusteranmeldung 20 2012 007 845.3 wird durch Bezugnahme aufgenommen.

Die Erfindung betrifft eine Katheterkupplung nach dem Oberbegriff des Anspruchs 1.

Eine derartige Katheterkupplung ist bekannt aus der EP 1 033 146 B1. Die DE 90 15 688 U1 offenbart eine Leitungsanordnung für die Verabreichung infundierbarer Mittel. Die DE 10 2007 029 229 A1 offenbart einen Spannadapter für einen Katheter. Die DE 84 25 441 U1 offenbart eine Verschließvorrichtung für ein in einen lebenden Körper einzuführendes rohrförmiges Element.

Es ist eine Aufgabe der vorliegenden Erfindung, die Anforderungen an eine Handhabung der Katheterkupplung zu reduzieren.

Diese Aufgabe ist erfindungsgemäß gelöst durch eine Katheterkupplung mit den im Anspruch 1 angegebenen Merkmalen.

Das Sichtfenster vermeidet, dass irrtümlich ein zu kurzer Endabschnitt des Katheters in das Schlauchstück eingeführt wird.

Die Katheterkupplung nach Anspruch 2 gehört zu einem weiteren Aspekt der Erfindung. Gemäß diesem weiteren Aspekt ist zwischen dem der Mündungsöffnung benachbarten Rast-Verriegelungselement und einem Kopfabschnitt der Kupplung, in dem die Mündungsöffnung ausgebildet ist, eine Aussparung angeordnet, die eine Relativbewegung des Kopfabschnitts zum Rast-Verriegelungselement ermöglicht. Aufgrund der eine Relativbewegung des Kopfabschnitts zum Rast-Verriegelungselement ermöglichenden Aussparung ist bei der Katheterkupplung gemäß diesem weiteren Aspekt eine Relativbewegung des Kopfabschnitts der Katheterkupplung zur Rast-Verriegelungseinrichtung unabhängig von einem Verschwenken der Backen zueinander über das Backen-Gelenk möglich. Ein Druck, der auf den Kopfabschnitt der Kupplung ausgeübt wird, führt dann nicht zu einer Verformung des Rast-Verriegelungselements. Die Rast-Verriegelungseinrichtung arbeitet auch dann, wenn ein solcher Druck ausgeübt wird, immer noch mit einer vorgegebenen Schließkraft. Dies erleichtert die Handhabung der Katheterkupplung. Eine Fehlbedienung, die zu einer Beeinträchtigung der Rast-Verriegelungseinrichtung führen kann, wird vermieden.

Die Katheterkupplung nach Anspruch 3 gehört zu einem weiteren Aspekt der Erfindung. Gemäß diesem Aspekt ist einer der Backen Teil eines Grundkörpers der Kupplung, der das Schlauchstück trägt, wobei der Grundkörper-Backen Schutzstege aufweist, zwischen denen das Schlauchstück versenkt angeordnet ist. Bei diesem Aspekt ist die Anzahl der voneinander unabhängigen Teile der Katheterkupplung gering. Bei aufgeklapptem Backen-Gelenk ist das Schlauchstück durch die Schutzstege gegen eine unerwünschte Verformung geschützt. Die Schutzstege können beiderseits des Schlauchstücks angeordnet sein. Bei den Schutzstegen kann es sich um durchgehende oder alternativ um durchbrochene Schutzstege handeln.

Die vorstehenden Aspekte können in Kombination mit den Merkmalen nach dem Oberbegriff des Anspruchs 1 jeweils für sich oder in beliebiger Kombination miteinander realisiert sein.

Eine Anordnung nach Anspruch 4 hat sich in der Praxis bewährt. Die Anzahl der voneinander unabhängigen Teile der Katheterkupplung ist gering.

Eine Zweikomponenten (2K)-Ausführung nach Anspruch 5 hat sich als besonders geeignet herausgestellt. Das weiche Schlauchstück lässt sich zur sichernden Aufnahme des Katheter-Endabschnitts einfach verformen. Insbesondere die mindestens eine Backen-Verformungskomponente ist aus härterem Kunststoff ausgeführt als das Schlauchstück.

Bei einer Ausführung nach Anspruch 6 ist bei aufgeklapptem Backen-Gelenk das Schlauchstück durch die Schutzstege gegen eine unerwünschte Verformung geschützt. Die Schutzstege können längs des Schlauchstücks verlaufen. Die Schutzstege können beiderseits des Schlauchstücks angeordnet sein. Bei den Schutzstegen kann es sich um durchgehende oder alternativ um durchbrochene Schutzstege handeln.

Ausnehmungen nach Anspruch 7 können zumindest bereichsweise komplementär zu den Schutzstegen ausgeführt sein. In diesem Fall können die Ausnehmungen beim Zusammenwirken mit den Schutzstegen auch eine Führungsfunktion einer Schwenkbewegung der Backen zueinander erfüllen.

Eine konkave Anlagefläche nach Anspruch 8 erleichtert ein definiertes Verformen des Schlauchstücks über die mindestens eine Backen-Verformungskomponente. Ein unerwünschtes Ausweichen des Schlauchstücks ist vermieden. Die konkave Krümmung der Anlagefläche kann um eine Achse parallel zur Längsachse des anliegenden Schlauchstücks verlaufen.

Eine Gestaltung nach Anspruch 9 hat sich zur sichernden Aufnahme des Katheter-Endabschnitts als geeignet und ausreichend herausgestellt. Alternativ kann eine durchgehend längs zumindest eines Abschnitts des Schlauchstücks verlaufende Backen-Verformungskomponente vorgesehen sein.

Ausführungsbeispiele der Erfindung werden nachfolgend anhand der Zeichnungen näher erläutert. In dieser zeigen:
- Fig. 1: perspektivisch eine Katheterkupplung mit einem Schlauchstück und zwei in Längsrichtung des Schlauchstücks verlaufenden Backen, die in einer aufgeklappten Stellung gezeigt sind;
- Fig. 2: eine Seitenansicht der Katheterkupplung mit den Backen in einer aufgeklappten Schrägstellung nach Fig. 1;
- Fig. 3: einen Querschnitt durch einen Gelenk-Backen gemäß Linie III-III in Fig. 2 in vergrößerter Darstellung;
- Fig. 4: eine perspektivische Ansicht der Katheterkupplung mit den Backen in einer zusammengeklappten Schließstellung;
- Fig. 5: aus einer zu Fig. 1 ähnlichen Blickrichtung eine perspektivische Darstellung einer weiteren Ausführung einer Katheterkupplung, ebenfalls mit aufgeklappten Backen.

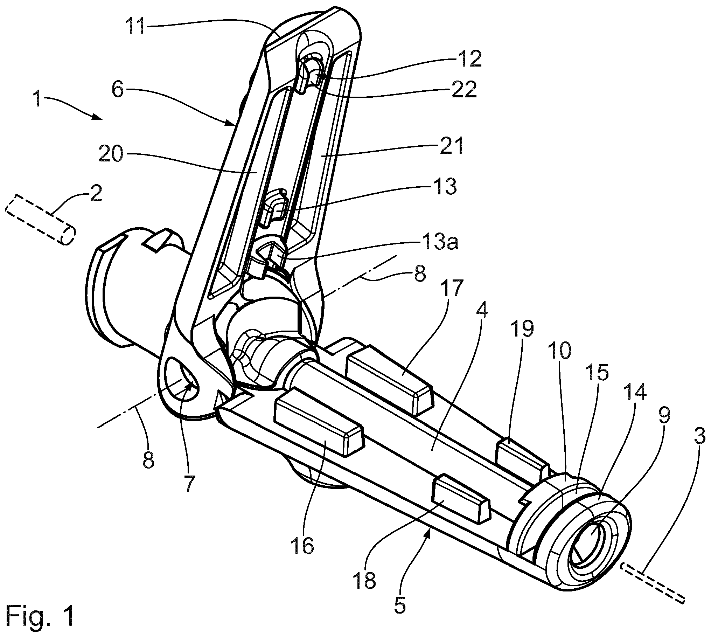

Eine Katheterkupplung 1 dient zur Fluidverbindung eines in der Fig. 1 gestrichelt dargestellten Schlauchs 2 mit einem Katheter 3, der in der Fig. 1 ebenfalls gestrichelt angedeutet ist.

Die Katheterkupplung hat ein Kupplungs-Schlauchstück 4 und zwei in Längsrichtung des Schlauchstücks verlaufende Backen, nämlich einen Grundkörper-Backen 5 und einen Gelenk-Backen 6.

Der Grundkörper-Backen 5 ist Teil eines Grundkörpers der Katheterkupplung 1, der das Schlauchstück 4 trägt.

Das Schlauchstück 4 ist einstückig mit dem Grundkörper-Backen 5 verbunden. Der Grundkörper ist in 2K-Technik ausgeführt, wobei das Schlauchstück 4 als Weichkomponente und der Grundkörper-Backen 5 als Hartkomponente ausgeführt ist.

Die beiden Backen 5,6 sind miteinander über ein Backen-Gelenk 7 mit einer Gelenkachse 8 quer zu einer Längserstreckung des Schlauchstücks 4 verbunden. Die Backen 5, 6 bilden in einer in der Zeichnung nicht dargestellten zusammengeklappten Stellung, also in aneinander längs des Schlauchstücks 4 anliegendem Zustand, mit dem Schlauchstück 4 einen Kanal mit einer Mündungsöffnung 9 zur Aufnahme eines Endabschnitts des Katheters 3.

Am jeweils vom Gelenk 7 abgewandten Ende haben die beiden Backen 5, 6 ein Verriegelungselement 10, 11. Das Rast-Verriegelungselement 10 des Grundkörper-Backens 5 ist als Rasthaken ausgebildet. Das Gegen-Rast-Verriegelungselement 11 des Gelenk-Backens 6 ist als Rastausnehmung ausgebildet, die zum Rasthaken 10 komplementär ausgeführt ist. Die beiden Verriegelungselemente bilden eine Rast-Verriegelungseinrichtung. In einer Raststellung der Rast-Verriegelungseinrichtung 10, 11, die bei vollständigem Einklappen des Gelenk-Backens 6 relativ zum Grundkörper-Backen 5 erreicht wird, wird das Schlauchstück 4 zur sichernden Aufnahme des Katheter-Endabschnitts über mindestens eine Backen-Verformungskomponente 12, 13 verformt. Die Backen-Verformungskomponenten 12, 13 sind einstückig an einer dem Grundkörper-Backen 5 zugewandten Seite am Gelenk-Backen 6 angeformt. Zusätzlich zu den Backen-Verformungskomponenten 12, 13 ist an den Gelenk-Backen 6 noch eine Positionssicherungskomponente 13a zur Sicherung einer korrekten Relativpositionierung einer Lage des Gelenk-Backens 6 zur Lage des Grundkörper-Backens 5 mit dem Schlauchstück 4 angeformt.

Zwischen dem der Mündungsöffnung 9 benachbarten Rast-Verriegelungselement 10 und einem Kopfabschnitt 14 der Katheterkupplung 1, in dem die Mündungsöffnung 9 ausgebildet ist, ist eine nutförmige Aussparung 15 angeordnet, die eine Relativbewegung des Kopfabschnitts 14 zum Rast-Verriegelungselement 10 ermöglicht. Beispielsweise ist es möglich, den Kopfabschnitt 14 auf das Rast-Verriegelungselement 10 zuzubewegen, wobei hierdurch die Aussparung 15 entsprechend leicht verengt wird. Diese Relativbewegung des Kopfelements 14 zum Rast-Verriegelungselement 10 ist unabhängig von einer Backenbewegung über das Backen-Gelenk 7 möglich. Die nutförmige Aussparung 15 verläuft quer zur Längsachse des Schlauchstücks 4 und bildet um dieses herum zwischen dem Kopfabschnitt 14 und dem Rast-Verriegelungselement 10 eine Halbkreis-Nut.

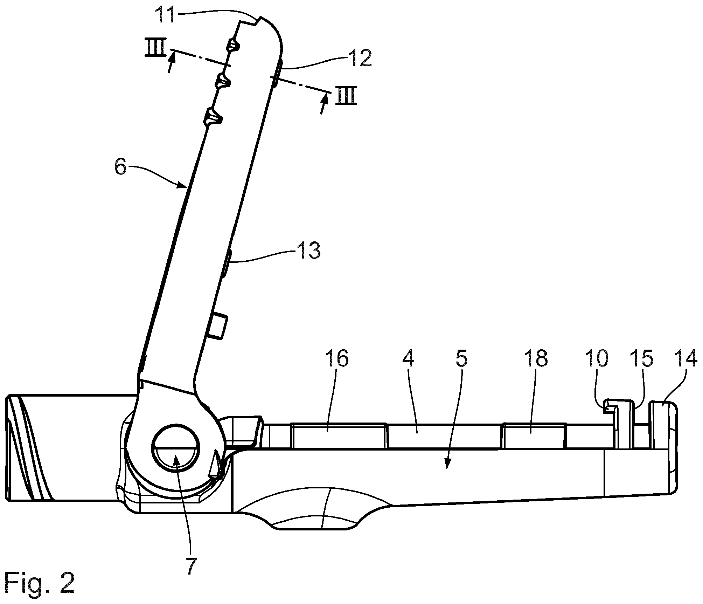

Der Grundkörper-Backen 5 weist Schutzstege 16, 17, 18, 19 auf, zwischen denen das Schlauchstück 4, wie aus der Seitenansicht der Fig. 2 deutlich wird, versenkt angeordnet ist. Bei aufgeklapptem Backen-Gelenk 6 ist das Schlauchstück 4 durch die Schutzstege 16 bis 19 gegen eine Verformung geschützt.

Die Schutzstege 16 bis 19 verlaufen jeweils längs des Schlauchstücks 4. Die Schutzstege 16 bis 19 sind einstückig am Grundkörper-Backen 5 angeformt. Die Schutzstege 16 bis 19 sind beiderseits des Schlauchstücks 4 angeordnet. Bei der Ausführung aus den Fig. 1 bis 3 liegt zwischen den jeweils auf einer Seite des Schlauchstücks 4 benachbart angeordneten Schutzstegen 16, 18 und 17, 19 eine Aussparung bzw. Unterbrechung vor.

Der Gelenk-Backen 6 hat Ausnehmungen 20, 21, in die bei aneinanderliegenden Backen 5, 6 die Schutzstege 16 bis 19 eingreifen. Die Ausnehmungen 20, 21 sind im Wesentlichen komplementär zu den Stegen 16 bis 19 ausgeführt, sodass sie in Bezug auf das Erreichen einer Sicherungsstellung der Backen 5, 6 zueinander, in der die Verriegelungselemente 10, 11 miteinander verriegelnd zusammenwirken und die Backen 5, 6 einander anliegen, eine Bewegung der Backen 5, 6 zueinander führen.

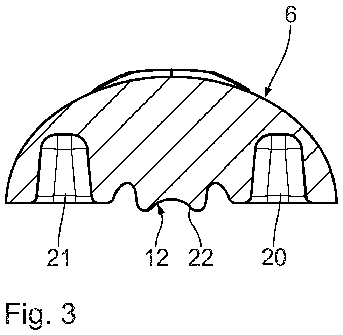

Die Backen-Verformungskomponenten, von denen die Backen-Verformungskomponente 12 in der Fig. 3 vergrößert im Querschnitt dargestellt ist, haben jeweils eine konkave Anlagefläche 22, die bei aneinander anliegenden Backen 5, 6 außen am Schlauchstück 4 zum Verformen von diesen anliegt. Die konkaven Anlageflächen 22 der Backen-Verformungskomponenten 12, 13 sind jeweils um eine Achse gekrümmt, die parallel zur Längsachse des Schlauchstücks 4 verläuft.

Die beiden Backen-Verformungskomponenten 12, 13 sind voneinander beabstandet. Die Backen-Verformungskomponente 12 ist in Längsrichtung des Gelenk-Backens 6 benachbart zum Gegen-Rast-Verriegelungselement 11 angeordnet. Die andere Backen-Verformungskomponente 13 ist zwischen der Backen-Verformungskomponente 12 und dem Backen-Gelenk 7 angeordnet.

Die Katheterkupplung 1 ist insgesamt aus Kunststoff.

Die Katheterkupplung 1 wird folgendermaßen verwendet:

Zunächst liegt die Katheterkupplung 1 in einer Offenstellung nach den Fig. 1 und 2 vor. In dieser Offenstellung kann der Katheter 3 durch die Mündungsöffnung 9 bis zu einem definierten Anschlag in das Schlauchstück 4 eingeschoben werden. Dieser Anschlag liegt benachbart zur Höhe des Backen-Gelenks 7.

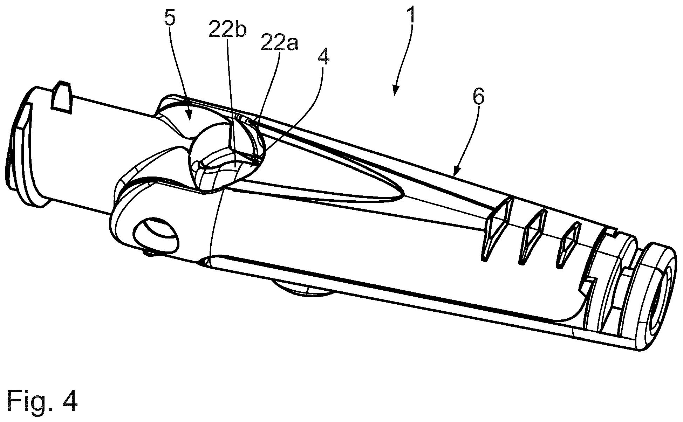

Im Grundkörper-Backen 5 kann im Bereich des Anschlages für den Endabschnitt des Katheters 3 ein transparenter Abschnitt bzw. ein Sichtfenster 22a angeordnet sein. Hierüber ist eine Sichtkontrolle möglich, ob der Endabschnitt des Katheters 3 den Anschlag erreicht hat. Fig. 4 zeigt die Katheterkupplung 1 mit geschlossenen Backen 5, 6, bei der das zwischen dem Gelenk-Backen 6 einerseits und dem Grundkörper-Backen 5 andererseits ausgebildete Sichtfenster 22a sichtbar ist. Zumindest ein Abschnitt 22b des Schlauchstücks 4 ist zumindest teiltransparent ausgeführt, so dass eine Position des Endabschnitts des Katheters 3 jedenfalls im Abschnitt 22b des Schlauchstücks 4 durch das Sichtfenster 22a per Sichtprüfung kontrolliert werden kann.

Nach dem vollständigen Einschieben des Katheters 3 in das Schlauchstück 4 bis zum Anschlag wird der Gelenk-Backen 6 um das Backen-Gelenk 7 verschwenkt, bis die Verriegelungselemente 10, 11 rastend miteinander zusammenwirken. Hierbei tauchen die Schutzstege 16 bis 19 in die Ausnehmungen 20, 21 ein. Bei verrasteten Backen 5, 6 verformen die Backen-Verformungskomponenten 12, 13 diejenigen Bereiche des Schlauchstücks 4, auf denen die Backen-Verformungskomponenten 12, 13 aufliegen. Durch diese Verformung dieser Abschnitte des Schlauchstücks 4 wird der Katheter 3 reibschlüssig im Schlauchstück 4 gesichert und ist auf diese Weise sicher mit der Katheterkupplung 1 verbunden. Über einen in den Fig. 1 bis 3 nicht dargestellten Luerlock-Anschluss ist die Katheterkupplung 1 mit dem Schlauch 2 zum Zuführen beispielsweise eines Medikaments, insbesondere eines Anästhetikums bei der Epiduralanästhesie, verbindbar.

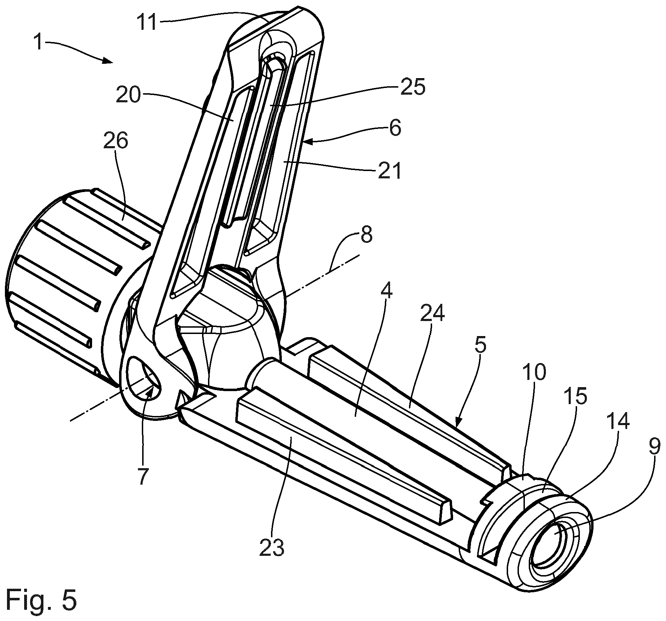

Fig. 5 zeigt in einer zu Fig. 1 ähnlichen Ansicht eine weitere Ausführung der Katheterkupplung 1. Komponenten und Funktionen, die denjenigen entsprechen, die vorstehend unter Bezugnahme auf die Fig. 1 bis 4 bereits erläutert wurden, tragen die gleichen Bezugsziffern und werden nicht nochmals im Einzelnen diskutiert.

Anstelle der Schutzstege 16, 17 und 18, 19 hat der Katheter 1 nach Fig. 5 zwei durchgehende Schutzstege 23, 24, deren Funktion den Schutzstegen 16 bis 19 entspricht.

Der Katheter 1 nach Fig. 5 hat zudem eine einzige Backen-Verformungskomponente 25, die sich längs etwa zwei Dritteln des Gelenk-Backens 6 erstreckt und den gleichen Querschnitt hat wie die Backen-Verformungskomponenten 12, 13 nach den Fig. 1 bis 4. Entsprechend wirkt die Backen-Verformungskomponente 25 längs ihrer gesamten Erstreckung längs des Schlauchstücks 4 auf dieses, sobald die Backen 5, 6 aneinander anliegen und rastend miteinander verbunden sind.

Bei der Katheterkupplung 1 nach Fig. 5 ist zudem noch ein Luerlock-Anschluss 26 dargestellt.

## Patentansprüche

1. Katheterkupplung (1) mit einem Grundkörper, mit einem Schlauchstück (4) und mit zwei in Längsrichtung des Schlauchstücks (4) verlaufenden Backen (5, 6), die in aneinanderliegendem Zustand mit dem Schlauchstück (4) einen Kanal mit einer Mündungsöffnung (9) zur Aufnahme eines Endabschnitts eines Katheters (3) bilden,
- wobei die Backen (5, 6) an jeweils einem Backenende miteinander über ein Backen-Gelenk (7) mit Gelenkachse (8) quer zum Schlauchstück (4) verbunden sind und am jeweiligen anderen Backenende ein Rast-Verriegelungselement (10) und ein hiermit zusammenwirkendes Gegen-Rast-Verriegelungselement (11) aufweisen, wobei die beiden Verriegelungselemente (10, 11) eine Rast-Verriegelungseinrichtung bilden,
- wobei in einer Raststellung der Rast-Verriegelungseinrichtung (10, 11) das Schlauchstück (4) zur sichernden Aufnahme des Katheter-Endabschnitts über wenigstens eine Backen-Verformungskomponente (12, 13; 25) verformt ist,
**gekennzeichnet durch** mindestens ein Sichtfenster (22a) in einem Grundkörper, über das eine Sichtkontrolle eines Anschlags im Schlauchstück (4) für den Endabschnitt des Katheters möglich ist.

2. Katheterkupplung nach Anspruch 1, **dadurch gekennzeichnet, dass** zwischen dem der Mündungsöffnung (9) benachbarten Rast-Verriegelungselement (10) und einem Kopfabschnitt (14) der Kupplung (1), in dem die Mündungsöffnung (9) ausgebildet ist, eine Aussparung (15) angeordnet ist, die eine Relativbewegung des Kopfabschnitts (14) zum Rast-Verriegelungselement (10) ermöglicht.

3. Katheterkupplung nach Anspruch 1 oder 2, **dadurch gekennzeichnet, dass** einer der Backen (5) Teil eines Grundkörpers der Kupplung (1) ist, der das Schlauchstück (4) trägt, wobei der Grundkörper-Backen (5) Schutzstege (16 bis 19; 23, 24) aufweist, zwischen denen das Schlauchstück (4) versenkt angeordnet ist.

4. Katheterkupplung nach Anspruch 1 oder 2, **dadurch gekennzeichnet, dass** einer der Backen (5) Teil eines Grundkörpers der Kupplung (1) ist, der das Schlauchstück (4) trägt.

5. Katheterkupplung nach Anspruch 3 oder 4, **dadurch gekennzeichnet, dass** das Schlauchstück (4) einstückig mit dem Grundkörper-Backen (5) verbunden ist, wobei das Schlauchstück (4) als Weichkomponente und der Backen (5) als Hartkomponente einer 2K-Komponente (4, 5) ausgeführt ist.

6. Katheterkupplung nach einem der Ansprüche 3, 4 oder 5, **dadurch gekennzeichnet, dass** der Grundkörper-Backen (5) Schutzstege (16 bis 19; 23, 24) aufweist, zwischen denen das Schlauchstück (4) versenkt angeordnet ist.

7. Katheterkupplung nach Anspruch 3 oder 6, **dadurch gekennzeichnet, dass** der gelenkig mit dem Grundkörper-Backen (5) verbundene Gelenk-Backen (6) Ausnehmungen (20, 21) aufweist, in die bei aneinanderliegenden Backen (5, 6) die Schutzstege (16 bis 19; 23, 24) eingreifen.

8. Katheterkupplung nach einem der Ansprüche 1 bis 7, **dadurch gekennzeichnet, dass** mindestens eine Backen-Verformungskomponente (12, 13; 25) eine konkave Anlagefläche (22) aufweist, die bei aneinander anliegenden Backen (5, 6) außen am Schlauchstück (4) anliegt.

9. Katheterkupplung nach einem der Ansprüche 1 bis 8, **gekennzeichnet durch** mindestens zwei Backen-Verformungskomponenten (12, 13), die in aneinanderliegendem Zustand der Backen (5, 6) längs des Schlauchstücks (4) voneinander beabstandet vorliegen.

## Claims

1. Catheter coupling (1) having a main body, having a tube piece (4), and having two jaws (5, 6) which extend in a longitudinal direction of the tube piece (4) and which, in a state of abutment with one another, form, together with the tube piece (4), a channel with a mouth opening (9) for receiving an end portion of a catheter (3),
- wherein the jaws (5, 6) are connected to one another at in each case one jaw end via an articulated jaw joint (7) with an articulated joint axis (8) transverse to the tube piece (4) and have at the respective other jaw end a latch locking element (10) and a mating latch locking element (11) which interacts with the latter, wherein the two locking elements (10, 11) form a latch locking device,
- wherein, in a latching position of the latch locking device (10, 11), the tube piece (4) is deformed via at least one jaw deformation component (12, 13; 25) for the purpose of receiving the catheter end portion in a securing manner,
**characterized by** at least one viewing window (22a) in a main body, via which viewing window it is possible to visually inspect a stop in the tube piece (4) for the end portion of the catheter.

2. Catheter coupling according to Claim 1, **characterized in that,** between the latch locking element (10), which is adjacent to the mouth opening (9), and a head portion (14) of the coupling (1), in which the mouth opening (9) is formed, there is arranged a recess (15) which allows a relative movement of the head portion (14) relative to the latch locking element (10).

3. Catheter coupling according to Claim 1 or 2, **characterized in that** one of the jaws (5) is part of a main body of the coupling (1) that bears the tube piece (4), wherein the main body jaw (5) has protective webs (16 to 19; 23, 24) between which the tube piece (4) is arranged in a sunken manner.

4. Catheter coupling according to Claim 1 or 2, **characterized in that** one of the jaws (5) is part of a main body of the coupling (1) that bears the tube piece (4).

5. Catheter coupling according to Claim 3 or 4, **characterized in that** the tube piece (4) is integrally connected to the main body jaw (5), wherein the tube piece (4) is designed as a soft component, and the jaw (5) is designed as a hard component, of a 2-component part (4, 5).

6. Catheter coupling according to one of Claims 3, 4 or 5, **characterized in that** the main body jaw (5) has protective webs (16 to 19; 23, 24) between which the tube piece (4) is arranged in a sunken manner.

7. Catheter coupling according to Claim 3 or 6, **characterized in that** the articulated joint jaw (6), which is connected to the main body jaw (5) in an articulated manner, has recesses (20, 21) into which the protective webs (16 to 19; 23, 24) engage when the jaws (5, 6) are in abutment with one another.

8. Catheter coupling according to one of Claims 1 to 7, **characterized in that** at least one jaw deformation component (12, 13; 25) has a concave abutment surface (22), which abuts with the outside of the tube piece (4) when the jaws (5, 6) are in abutment with one another.

9. Catheter coupling according to one of Claims 1 to 8, **characterized by** at least two jaw deformation components (12, 13) which, in a state of abutment of the jaws (5, 6) with one another, are spaced apart from one another along the tube piece (4).

## Revendications

1. Raccord pour cathéter (1) comportant un corps de base, un morceau de tuyau (4) et deux mâchoires (5, 6) qui s'étendent dans la direction longitudinale du morceau de tuyau (4) et qui, lorsqu'elles reposent l'une contre l'autre, forment avec le morceau de tuyau (4) un canal comportant une ouverture d'embouchure (9) destinée à recevoir une partie d'extrémité d'un cathéter (3),
- les mâchoires (5, 6) étant reliées l'une à l'autre à une extrémité de mâchoire par l'intermédiaire d'une articulation de mâchoire (7) comportant un axe d'articulation (8) transversalement par rapport au morceau de tuyau (4), et comportant, à l'autre extrémité de mâchoire respective, un élément de verrouillage à encliquetage (10) et un élément de verrouillage à encliquetage antagoniste (11) coopérant avec celui-ci, les deux éléments de verrouillage (10, 11) formant un dispositif de verrouillage à encliquetage,
- le morceau de tuyau (4) étant déformé pour recevoir de manière sûre la partie d'extrémité du cathéter via au moins une composante de déformation de mâchoire (12, 13 ; 25) dans une position d'encliquetage du dispositif de verrouillage à encliquetage (10, 11),
**caractérisé par** au moins une fenêtre de visualisation (22a) dans un corps de base, à travers laquelle il est possible d'inspecter visuellement une butée dans le morceau de tuyau (4) de la partie d'extrémité du cathéter.

2. Raccord pour cathéter selon la revendication 1, **caractérisé en ce qu'**entre l'élément de verrouillage à encliquetage (10) adjacent à l'ouverture d'embouchure (9) et une partie de tête (14) du raccord (1), dans laquelle se forme l'ouverture d'embouchure (9), se situe un évidement (15) qui permet un mouvement relatif de la partie de tête (14) par rapport à l'élément de verrouillage à encliquetage (10).

3. Raccord pour cathéter selon la revendication 1 ou 2, **caractérisé en ce que** l'une des mâchoires (5) fait partie d'un corps de base du raccord (1) qui porte le morceau de tuyau (4), la mâchoire du corps de base (5) étant pourvue de barres de protection (16 à 19 ; 23, 24) entre lesquelles le morceau de tuyau (4) est encastré.

4. Raccord pour cathéter selon la revendication 1 ou 2, **caractérisé en ce que** l'une des mâchoires (5) fait partie d'un corps de base du raccord (1) qui porte le morceau de tuyau (4).

5. Raccord pour cathéter selon la revendication 3 ou 4, **caractérisé en ce que** le morceau de tuyau (4) est relié d'un seul tenant à la mâchoire du corps de base (5), le morceau de tuyau (4) étant conçu comme une composante souple et la mâchoire (5) comme une composante dure d'une composante 2K (4, 5).

6. Raccord pour cathéter selon l'une des revendications 3, 4 ou 5, **caractérisé en ce que** la mâchoire du corps de base (5) est pourvue de barres de protection (16 à 19 ; 23, 24) entre lesquelles le morceau de tuyau (4) est encastré.

7. Raccord pour cathéter selon la revendication 3 ou 6, **caractérisé en ce que** la mâchoire articulée (6) reliée de manière articulée à la mâchoire du corps de base (5) présente des évidements (20, 21) dans lesquels les barres de protection (16 à 19 ; 23, 24) s'engagent lorsque les mâchoires (5, 6) reposent l'une contre l'autre.

8. Raccord pour cathéter selon l'une des revendications 1 à 7, **caractérisé en ce qu'**au moins une composante de déformation de mâchoire (12, 13 ; 25) présente une surface de contact concave (22) qui, lorsque les mâchoires (5, 6) reposent l'une contre l'autre, repose contre l'extérieur du morceau de tuyau (4).

9. Raccord pour cathéter selon l'une des revendications 1 à 8, **caractérisé par** au moins deux composantes de déformation de mâchoires (12, 13) qui, lorsque les mâchoires (5, 6) reposent l'une contre l'autre, se trouvent à distance l'une de l'autre le long du morceau de tuyau (4).
